# EUROPEAN PATENT SPECIFICATION

(11) **EP 1 411 877 B1**
(45) Date of publication and mention of the grant of the patent: **14.12.2005**
(21) Application number: 02741617.1
(22) Date of filing: 17.07.2002
(51) Int. Cl.: A61F 13/15, A61F 13/64

(54) **ABSORBENT ARTICLE**
SAUGFÄHIGER ARTIKEL
ARTICLE ABSORBANT

(30) Priority: 17.07.2001 SE 0102553
(43) Date of publication of application: 28.04.2004
(73) Proprietor: SCA Hygiene Products AB, 405 03 Göteborg (SE)
(72) Inventor: KUSIBOJOSKA, Liljana, S-254 38 Helsingborg (SE); ALMBERG, Christian, S-435 41 Mölnlycke (SE)
(74) Representative: Egeröd, Lisbeth
(86) International application number: PCT/SE2002/001393
(87) International publication number: WO 2003/007863

(56) References cited:
- GB-A- 1 200 177
- US-A- 2 224 518
- US-A- 3 618 608
- US-A- 3 890 973
- US-A- 4 604 096
- US-A- 4 617 022

## Description

### Technical field

The present invention relates to an absorbent article such as a diaper and an incontinence guard comprising a liquid permeable topsheet, a liquid impervious backsheet and an absorbent body enclosed therebetween, whereby the article seen in longitudinal direction exhibits a front portion, a rear portion and a crotch portion arranged therebetween, and further exhibits a first belt portion and a second belt portion, which belt portions are attached to the rear portion alternatively the front portion of the article and where first fastening means is attached on the second belt portion and is intended to be attached against the first belt portion around the waist of the user and where said front portion alternatively the rear portion exhibits second fastening means intended to be attached against the belt portions in such a way that the article assumes a pantlike shape where the belt portions form a part of the waist portions of the pant.

### Background of the invention

Diapers and incontinence guards for incontinent adults usually exhibits a garment portion holding an absorbent body in place against the users body and fastening means which hold the garment portion in place also when the user is moving. A common type of fastening means are adhesive tapes or hook and loop fasteners of the touch and close type, which directly attach the front and rear portions of the absorbent article to each other. It is further known, through e.g., EP-A-0 287 388, EP-A-0 409 307, EP-A-0 528 282, EP-A-0 605 012 and FR-A-2 586 558, to attach the front and rear portions of the article by means of a belt, at which the possibilities to adjust the fit are improved. The belt further provides a simplified change of diaper or incontinence guard, especially when the patient is standing up.

On a common type of belt diaper, the belt portions are first attached around the waist of the patient and then the front portion of the diaper is attached to the outside of the belt using hook and loop fasteners, whose hook member being arranged on the front portion of the article, whereby the outside of the belt functions as a reception surface or the loop member of the hook and loop type fastener. One problem is that a belt of this kind has a limited flexibility regarding the possibilities to adjust its length. The belt portions are usually attached using hook and loop type fastener to each other. The backsheet material is usually a plastic film. If the belt is applied on a person having a small waist size, then the belt portions will reach around the patient all the way to the material on the rear portions of the diaper, where the hook and loop fasteners can not attach. This leads to a poor fit. For a medium sized article to for instance fit on a small sized person, who then exhibits a smaller waist size, a belt is required that allows an improved regulation of the waist size. The same applies to adapt the size of a large sized article on an extra large person, where the belt portions do not reach around the waist of the person in question. This would be of great importance on hospitals and in elderly care, since a smaller number of sizes of the articles could be used on as many patients as possible.

### Summary of the invention

The object of the present invention is to provide a diaper or incontinence guard, which can be used on persons having very different waist sizes. This object is being solved in that the first belt portion, whose outside constitutes reception surface for said first fastening means, is at least 10 % longer than the second belt portion, carrying said first fastening means.

This design leads to, since the reception surface for the first fastening means has been made longer than the length of the belt portion carrying the first fastening means, an improved possibility to adjust the waist size of the article to the size of the user, whereby a belted article of a certain size can be used on more persons.

### Brief description of the drawings

The invention will in the following be closer described with reference to an embodiment shown in the accompanying drawing.

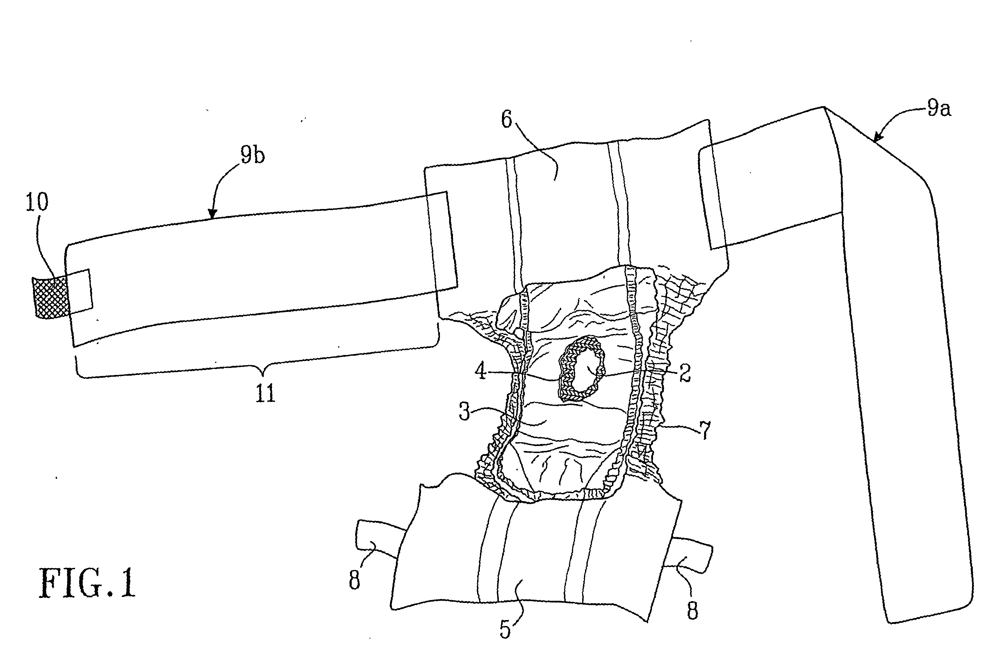

Fig. 1 shows schematically a perspective view of a diaper or incontinence guard according to the invention.

### Detailed description of the invention

Fig. 1 shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material, which resists liquid penetration. The backsheet material 2 and the topsheet 3 have a somewhat greater extension in the plane than the absorbent body 4 and extend outside the edges thereof. The layers 2 and 3 are connected to each other within the projecting portions thereof, e.g., by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well known to the person skilled in the art and does therefore not have to be described in detail. The thin absorbent bodies that are common in for example baby diapers and incontinence guards often comprise a compressed mixed or layered structure of cellulosic fluff pulp and superabsorbent.

The diaper incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs.

A pair of belt portions 9a, 9b are with one end attached, e.g., glued or ultrasonically welded, to the rear portion 6 of the diaper. The belt portions 9a, 9b are with their opposite ends intended to be fastened together by means of first fastening means 10. The fastening means 10 may be a hook-and-loop type fasteners or tape tabs. The outside portions of the belt portions 9a, 9b comprise reception surface for the fastening means 10. The belt portions 9a, 9b exhibit on the inside, which is intended to be faced against the body, a soft, skin friendly material, preferably a nonwoven material. The fastening means 10 is preferably located on the right belt portion 9b. The location of the fastening means 10 may naturally be the other way around, i.e., that the left belt portion carries the fastening means 10. However, since most people are right handed, are the embodiments described above and below probably the most preferred.

The definition hook and loop type fastener denotes a mechanical fastening device, where one part is a surface comprising a hook material and one part is a reception surface having a loop material. The loop material preferably consists of a nonwoven material. The term "hook material" shall not be regarded as being limited to a material having hooks, but comprises all materials having different shapes that are capable to mechanically attach to complementary reception surfaces. The term "loop material" shall not be regarded as being limited to a material having separately formed loops and having the capability to attach to the hook members of a complementary hook material, but also comprise fibrous nonwoven material, in which the hook members from the complementary hook material surface can attach to the separate fibres without these fibres being shaped into separate loops.

The fastening means 8 of the front portion 5 is intended to be attached against the outsides of the belt portions 9a, 9b, in order to fasten together the diaper/incontinence guard to the desired pantlike shape. The fastening means 8 can be hook and loop type fasteners or tape tabs. According to an alternative embodiment, the belt portions 9a, 9b are attached to the front portion 5 of the diaper and thus are intended to be fastened together on the back of the wearer. The fastening means 8 are then arranged on the rear portion 6 of the diaper.

The width of the belt portions 9a, 9b should be between 5-20 cm, preferably between 7-15 cm. The belt portions 9 are preferably a laminate of a carrier material, which forms the outside of the belt, and a soft nonwoven material, which forms the inside of the belt, which is intended to be in direct contact with the skin of the user. A suitable nonwoven material for the inside of the belt can be a spunbond material of e.g. polypropylene- or polyethylene fibres. Conjugate fibres may also be used. Another suitable nonwoven material can be a carded thermobonded material of e.g., polypropylene-, polyester- or conjugate fibres. As carrier material can for instance nonwoven or another suitable material be used. The carrier material should be adapted to function as a reception surface for both the attachment means 8 and 10. Also elastic laminates are suitable to use as material in the belt portions.

According to the present invention, the first belt portion 9a, preferably the left belt portion 9a is at least 10 %, preferably at least 15 % and most preferred at least 20 % longer than the other belt portion 9b. The lengths 11 of the belt portions are effective lengths, i.e., the length 11 is calculated from the attachment portion of the rear portion 6. When a diaper is applied on a user, firstly, the second belt portion 9b is brought to overlap the first belt portion 9a. Then the fastening means 10, i.e., the hook member 10, being arranged on the shorter belt portion 9b, is attached to the reception surface being on the outside of the belt portion 9a.

In one embodiment for an incontinence product intended for an adult wearer, the length of the first belt portion 9a may vary between 40 and 60 cm, preferably between 45 and 55 cm. The length of the other belt portion 9b may vary between 35 and 50 cm, preferably between 38 and 45 cm. When a large sized diaper is applied on a user having an extra large waist size, firstly, the first belt portion is brought to overlap the second belt portion 9b over the first belt portion 9a. The fastening means 10, being arranged on the shorter second belt portion 9b then reaches all the way to and attaches to the extended reception surface for the fastening means 10, which is arranged on the outside of the belt portion 9a. If the belt portions 9a, 9b have been equally long, then the belt portion carrying the fastening means 10, had not reached the reception surface for the fastening means 10, which is arranged on the outside of the belt portion 9a and had thereby not been able to attach to this belt portion 9a. According to the invention the reception surface for the fastening means 10 has been extended since the second belt portion 9a has been made longer, whereby the possibility to increase the waist size of the diaper has been increased. A large sized diaper can in this way be adapted to fit an extra large sized person. In this embodiment the fastening means 10 can be hook and loop type fasteners or tape tabs.

In a second embodiment is the first belt portion 9a at least 50 % longer than the second belt portion 9b. The length of the first belt portion 9a for an incontinence product intended for an adult wearer may vary between 35 and 55 cm preferably between 40 and 50 cm. The length of the other belt portion 9b may vary between 15 and 35 cm, preferably between 20 and 30 cm. When the user has a small waist size, the belt portion 9b carrying fastening means 10, would reach all the way to the backsheet material 2 on the rear portion 6 of the article, if the belt portions 9a, 9b had been equally long. As the backsheet material 2 usually is a plastic film, the fastening means 10, being a hook member to a hook and loop type fastener, could not attach to this kind of backsheet 2. According to the invention, the first belt portion 9a has been made longer than the second belt portion 9b, whereby the possibilities to decrease the waist size of the diaper has been increased. A medium sized diaper can in this way be adapted to fit a small sized person.

If the design or the proportions of the rear portion 6 would differ from the embodiment shown in Fig. 1, the lengths of the belt portion could then be estimated from a centre point on the rear portion 6. The length of the belt portion 9a calculated from the centre point on the rear portion 6 of the article, is then about 52-76 cm and the length of the belt portion 9b is then 32-56 cm for the smaller size. For the larger size, the length is 47-81 cm for the belt portion 9a calculated from the centre point on the rear portion 6 of the article and the length of the belt portion 9b is 52-71 cm. If the belt portions according to the present invention were used on an article provided with a belt for children, then the article would have to be adapted to suitable proportions and lengths for a child. The above given lengths can in on line production deviate about +/- 0,5 cm from the given values. The invention is also intended to include such differences.

The diaper may now be adapted to different persons depending on their size with remained fit. It leads to a higher flexibility on e.g., hospitals and homes for elderly people, when the same diaper size may be used to an increased number of persons.

The invention is of course not limited to the above described and on the drawing showed embodiment but can be modified within the scope of the claims.

## Claims

1. Absorbent article such as a diaper and an incontinence guard comprising a liquid permeable topsheet (3), a liquid impervious backsheet (2) and an absorbent body (4) enclosed there between, whereby the article seen in longitudinal direction exhibits a front portion (5), a rear portion (6) and a crotch portion (7) arranged therebetween, and further exhibits a first belt portion (9a) and a second belt portion (9b), which belt portions (9a, 9b) are attached to the rear portion (6) alternatively the front portion (5) of the article and where first fastening means (10) is attached on the second belt portion (9b) and is attachable against the first belt portion (9a) around the waist of the user and where said front portion (5) alternatively the rear portion (6) exhibits second fastening means (8), which are attachable against the belt portions (9a, 9b) in such a way that the article assumes a pantlike shape where the belt portions (9a, 9b) form a part of the waist portions of the pant,
**characterised in,**
**that** the first belt portion (9a), whose outside constitutes reception surface for said first fastening means (10), is at least 10 % longer than the second belt portion (9b), carrying said first fastening means (10).

2. Absorbent article according to claim 1,
**characterised in,**
**that** the first belt portion (9a) is at least 15 %, preferably at least 20 % longer than the second belt portion (9b).

3. Absorbent article according to claim 1,
**characterised in,**
**that** the first belt portion (9a) is at least 30 %, preferably at least 50 % longer than the second belt portion (9b).

4. Absorbent article according to claim 1,
**characterised in,**
**that** the first belt portion (9a) is at least 5 cm longer than the second belt portion (9b).

5. Absorbent article according to claim 1,
**characterised in,**
**that** the first belt portion (9a) is at least 8 cm, preferably at least 10 cm longer than the second belt portion (9b).

6. Absorbent article according to claim 1,
**characterised in,**
**that** the fastening means (10) consists of a hook member to a hook and loop type of fastener having the ability to attach to a reception surface arranged on the first belt portion (9a).

## Patentansprüche

1. Absorbierender Gegenstand wie beispielsweise eine Windel und ein Inkontinenzschutz, umfassend eine flüssigkeitsdurchlässige Oberlage (3), eine flüssigkeitsundurchlässige Rückseitenlagen (2) und einen dazwischen eingeschlossenen Absorptionskörper (4), wobei der Gegenstand in Längsrichtung einen Vorderabschnitt (5), einen Hinterabschnitt (6) und einen dazwischen angeordneten Schrittabschnitt (7) aufweist und ferner einen ersten Gürtelabschnitt (9a) und einen zweiten Gürtelabschnitt (9b) umfasst, die an dem Hinterabschnitt (6), alternativ dem Vorderabschnitt (5) des Gegenstandes angebracht sind und wobei eine erste Befestigungseinrichtung (10) an dem zweiten Gürtelabschnitt (9b) angebracht ist und gegen den ersten Gürtelabschnitt (9a) um die Taille des Verwenders anbringbar ist und wobei der Vorderabschnitt (5), alternativ der Hinterabschnitt (6), eine zweite Befestigungseinrichtung (8) aufweist, die gegen die Gürtelabschnitte (9a, 9b) derart anbringbar ist, dass der Gegenstand eine höschenähnliche Form annimmt, wobei die Gürtelabschnitte (9a, 9b) einen Teil der Taillenabschnitte des Höschens bilden,
**dadurch gekennzeichnet, dass**
der erste Gürtelabschnitt (9a), dessen Außenseite eine Aufnahmefläche für die erste Befestigungseinrichtung (10) bildet, wenigstens 10% länger ist als der zweite Gürtelabschnitt (9b), der die erste Befestigungseinrichtung (10) trägt.

2. Absorbierender Gegenstand nach Anspruch 1, **dadurch gekennzeichnet, dass** der erste Gürtelabschnitt (9a) wenigstens 15%, vorzugsweise wenigstens 20% länger ist als der zweite Gürtelabschnitt (9b).

3. Absorbierender Gegenstand nach Anspruch 1, **dadurch gekennzeichnet, dass** der erste Gürtelabschnitt (9a) wenigstens 30%, vorzugsweise wenigstens 50% länger ist als der zweite Gürtelabschnitt (9b).

4. Absorbierender Gegenstand nach Anspruch 1, **dadurch gekennzeichnet, dass** der erste Gürtelabschnitt (9a) wenigstens 5 cm länger ist als der zweite Gürtelabschnitt (9b).

5. Absorbierender Gegenstand nach Anspruch 1, **dadurch gekennzeichnet, dass** der erste Gürtelabschnitt (9a) wenigstens 8 cm, vorzugsweise wenigstens 10 cm länger ist als der zweite Gürtelabschnitt (9b).

6. Absorbierender Gegenstand nach Anspruch 1, **dadurch gekennzeichnet, dass** die Befestigungseinrichtung (10) aus einem Hakenelement einer klettverschlussartigen Befestigungseinrichtung besteht, das die Fähigkeit aufweist, an der Aufnahmefläche, die auf dem ersten Gürtelabschnitt (9a) angeordnet ist, anzuhaften.

## Revendications

1. Article absorbant tel qu'une couche-culotte ou une protection contre l'incontinence comprenant une feuille avant (3) perméable aux liquides, une feuille arrière (2) imperméable aux liquides et un corps absorbant (4) enfermé entre elles, l'article, vu dans sa direction longitudinale, présentant une partie avant (5), une partie arrière (6) et une partie entrejambe (7) située entre elles, et présentant en outre une première partie de ceinture (9a) et une deuxième partie de ceinture (9b), lesquelles parties de ceinture (9a, 9b) sont fixées à la partie arrière (6) alternativement à la partie avant (5) de l'article et où un premier moyen de fermeture (10) est fixé sur la deuxième partie de ceinture (9b) et peut être attaché contre la première partie de ceinture (9a) autour de la taille de l'utilisateur et où ladite partie avant (5) alternativement la partie arrière (6) présente des deuxièmes moyens de fermeture (8), qui peuvent être attachés contre les parties de ceinture (9a, 9b) de telle manière que l'article prend une forme de culotte où les parties de ceinture (9a, 9b) forment une partie des parties de taille de la culotte,
**caractérisé en ce que** la première partie de ceinture (9a), dont l'extérieur constitue une surface de réception pour ledit premier moyen de fermeture (10), est au moins 10 % plus longue que la deuxième partie de ceinture (9b), portant ledit premier moyen de fermeture (10).

2. Article absorbant selon la revendication 1, **caractérisé en ce que** la première partie de ceinture (9a) est au moins 15 %, de préférence au moins 20 %, plus longue que la deuxième partie de ceinture (9b).

3. Article absorbant selon la revendication 1, **caractérisé en ce que** la première partie de ceinture (9a) est au moins 30 %, de préférence au moins 50 %, plus longue que la deuxième partie de ceinture (9b).

4. Article absorbant selon la revendication 1, **caractérisé en ce que** la première partie de ceinture (9a) est plus longue que la deuxième partie de ceinture (9b) d'au moins 5 cm.

5. Article absorbant selon la revendication 1, **caractérisé en ce que** la première partie de ceinture (9a) est plus longue que la deuxième partie de ceinture (9b) d'au moins 8 cm, et de préférence d'au moins 10 cm.

6. Article absorbant selon la revendication 1, **caractérisé en ce que** le moyen de fermeture (10) est constitué d'un élément à crochets d'une fermeture du type à crochets et à boucles ayant la capacité de se fixer à une surface de réception placée sur la première partie de ceinture (9a).
